# FASCICULE DE BREVET EUROPEEN

(11) **EP 1 267 881 B1**
(45) Date de publication et mention de la délivrance du brevet: **31.08.2005**
(21) Numéro de dépôt: 01919589.0
(22) Date de dépôt: 28.03.2001
(51) Int. Cl.: A61K 31/555, A61K 31/409, A61K 31/00, A61P 1/16

(54) **UTILISATION DE MnTBAP DANS LE TRAITEMENT D'INSUFFISANCES HEPATOCELLULAIRES**
VERWENDUNG VON MnTBAP ZUR BEHANDLUNG VON HEPATISCHER INSUFFIZIENZ
USE OF MnTBAP FOR TREATING HEPATOCELLULAR INSUFFICIENCIES

(30) Priorité: 28.03.2000 FR 0003887
(43) Date de publication de la demande: 02.01.2003
(73) Titulaire: UNIVERSITE RENE DESCARTES, (PARIS V), F-75006 Paris (FR)
(72) Inventeur: WEILL, Bernard, 95600 Eaubonne (FR); BATTEUX, Frédéric, 75015 Paris (FR); FERRET, Pierre-Jacques, 92100 Boulogne-Billancourt (FR)
(74) Mandataire: Vialle-Presles, Marie José
(86) Numéro de dépôt international: PCT/FR2001/000939
(87) Numéro de publication internationale: WO 2001/072327

(56) Documents cités:
- EP-A- 0 289 667
- EP-A- 0 333 490
- EP-A- 0 676 472
- WO-A-00/19993
- WO-A-00/43395
- WO-A-94/15925
- WO-A-95/10185
- WO-A-95/31197
- WO-A-99/23097
- US-A- 5 834 509
- US-A- 5 874 421
- MELOV S ET AL: "Superoxide and mitochondria: Consequences and potential therapies." ENVIRONMENTAL AND MOLECULAR MUTAGENESIS, vol. 31, no. SUPPL. 29, 1998, page 23 XP000974813 29th Annual Meeting of the Environmental Mutagen Society;Anaheim, California, USA; March 21-26, 1998 ISSN: 0893-6692
- KITAHARA JUN ET AL: "Possible involvement of active oxygen species in selenite toxicity in isolated rat hepatocytes." ARCHIVES OF TOXICOLOGY, vol. 67, no. 7, 1993, pages 497-501, XP001030480 ISSN: 0340-5761
- MIESEL R ET AL: "Hepatoprotective reactivity of a copper-di-Schiffbase active centre analogue of Cu2Zn2 superoxide dismutase." GENERAL PHARMACOLOGY, (1995 OCT) 26 (6) 1261-6., XP000972539
- MIESEL R ET AL: "Antiinflammatory reactivity of copper(I)-thionein." INFLAMMATION, (1990 OCT) 14 (5) 471-83., XP000972526
- DATABASE WPI Week 199210 Derwent Publications Ltd., London, GB; AN 1992-076278 XP002155101 HASHIDA MITSURU: "SOD derivative" & JP 04 020285 A (TOYO JOZO CO LTD), 23 janvier 1992 (1992-01-23)
- DATABASE WPI Week 198920 Derwent Publications Ltd., London, GB; AN 1989-147379 XP002155102 KANBE TOSHIZANE: "Pharmaceutical preparations for oral administration" & JP 01 090133 A (OTSUKA PHARM CO LTD), 6 avril 1989 (1989-04-06) & PATENT ABSTRACTS OF JAPAN vol. 013, no. 302, 12 juillet 1989 (1989-07-12) & JP 01 090133 A (OTSUKA PHARM CO LTD)
- WENDEL A ET AL: "Evidence for the involvement of a reperfusion injury in galactosamine/endotoxin-induced hepatitis in mice." BIOCHEMICAL PHARMACOLOGY, (1987 AUG 15) 36 (16) 2637-9., XP000972711
- ZHANG ZILI ET AL: "Prolonged ethanol treatment enhances lipopolysaccharide/phorbol myristate acetate-induced tumor necrosis factor-alpha production in human monocytic cells." ALCOHOLISM CLINICAL AND EXPERIMENTAL RESEARCH, vol. 25, no. 3, mars 2001 (2001-03), pages 444-449, XP001030481 ISSN: 0145-6008
- FERRET PIERRE-JACQUES ET AL: "Detoxification of reactive oxygen species by a nonpeptidyl mimic of superoxide dismutase cures acetaminophen-induced acute liver failure in the mouse." HEPATOLOGY, vol. 33, no. 5, mai 2001 (2001-05), pages 1173-1180, XP001030468 ISSN: 0270-9139

## Description

L'invention est relative à de nouvelles utilisations de mimétiques de la superoxyde dismutase, dans le cadre du traitement des insuffisances hépatocellulaires.

On désigne sous le terme d'insuffisance hépatocellulaire un ensemble de manifestations pathologiques résultant de la destruction des hépatocytes. Selon l'étendue de la destruction cellulaire, ces manifestations cliniques sont plus ou moins graves et réversibles. Dans des cas extrêmes, la destruction massive et subite des hépatocytes aboutit à une insuffisance hépatique aiguë, également dénommée hépatite fulminante, qui peut entraîner la mort en quelques jours.

Parmi les causes les plus fréquentes de destruction des hépatocytes pouvant entraîner une insuffisance hépatocellulaire on mentionnera notamment les infections virales, dues aux différents types de virus de l'hépatite, ainsi que les intoxications, notamment par certains médicaments ou par l'alcool.

On dispose actuellement de plusieurs modèles expérimentaux animaux d'insuffisance hépatocellulaire de différentes origines, qui permettent notamment d'induire expérimentalement une insuffisance hépatique aiguë, et d'étudier les mécanismes aboutissant à la destruction cellulaire. Selon le facteur initial responsable de la destruction cellulaire, différents mécanismes ont ainsi été proposés.

Par exemple, l'insuffisance hépatique aiguë d'origine toxique, induite notamment par l'acétaminophène, résulte d'une saturation des mécanismes normaux de détoxication hépatique. En effet, aux doses pharmacologiques, l'aéétaminophène est principalement éliminé par glucoro- et sulfo-conjugaison, mais est également oxydé par le cytochrome P450 en N-acétyl-p-benziquinone-imine (NAPQI), qui peut normalement être ensuite éliminée après conjugaison avec le glutathion. En cas de surdosage, on observe la saturation des voies de glucoro- et sulfo-conjugaison, et une production accrue de NAPQI [PRESCOTT, Drugs, 25, 290-314, (1983)]. Ce métabolite très réactif est supposé être l'effecteur principal des lésions des hépatocytes, et les traitements médicamenteux proposés sont basés essentiellement sur l'utilisation d'antioxydants, tels que la N-acétyl-L-cystéine qui, ont pour but de permettre la reconstitution des réserves intracellulaires de glutathion et la neutralisation du NAPQI. L'efficacité de ces traitements est toutefois inconstante [CARACENI et VAN THIEL, Lancet, 345, 163-169, (1995) ; SCHIODT et al., N. Engl. J. Med., 337, 1112-1117, (1997)], et en cas d'hépatite fulminante, la greffe du foie constitue actuellement le seul traitement réellement efficace.

Le mécanisme proposé dans le cas de l'insuffisance hépatocellulaire d'origine virale est différent ; il implique principalement l'apoptose induite par l'interaction entre le récepteur Fas et son ligand (Fas/FasL). En conditions physiologiques, l'induction de l'apoptose Fas/FasL participe à la défense anti-virale, en permettant la destruction par les lymphocytes T cytotoxiques des cellules infectées exprimant l'antigène viral. Une amplification exagérée de ce phénomène entraîne une destruction massive des hépatocytes, qui, si elle intervient brutalement, peut prendre la forme d'une hépatite fulminante.

Dans le cas de l'insuffisance hépatocellulaire induite par l'alcool plusieurs mécanismes ont été proposés, faisant intervenir notamment l'apoptose médiée par les récepteurs de mort, ainsi que la génération de métabolites toxiques comme l'acétaldéhyde.

Les Inventeurs ont maintenant constaté que le MnTBAP, un mimétique de la superoxyde dismutase à manganèse (Mn-SOD) protégeait très efficacement les hépatocytes des effets destructeurs de substances toxiques ou d'infections virales, et possédaient ainsi un effet à la fois préventif et curatif de l'insuffisance hépatocellulaire, qu'elle soit aiguë ou chronique, et qu'elle soit d'origine virale ou toxique (médicamenteuse ou induite par l'alcool).

On désigne par le terme de superoxyde dismutase (SOD) une famille de métalloprotéines (EC 1.15.1.1), dont l'activité intervient dans la détoxification des radicaux oxygénés libres, en catalysant la dismutation de l'anion superoxyde (O.⁻²) en peroxyde d'hydrogène (H₂O₂). On distingue différents types de superoxyde dismutase, parmi lesquels on citera notamment les superoxyde dismutases à cuivre et zinc (CuZnSOD), également connues sous la désignation superoxyde dismutase-1, qui, chez les organismes eucaryotes, sont principalement localisées dans le cytoplasme, et les superoxyde dismutases à manganèse (MnSOD), également connues sous la désignation superoxyde dismutase-2, que l'on trouve principalement chez les procaryotes et dans les organites intracellulaires des cellules eucaryotes.

Le peroxyde d'hydrogène, généré par l'activité SOD, qui est lui-même toxique et peut générer d'autres radicaux oxygénés libres, est ensuite détoxifié par l'intermédiaire de diverses enzymes, notamment la catalase, la glutathion-peroxydase, et la thiorédoxine.

Le rôle joué par les radicaux oxygénés libres dans l'apparition et le développement de cancers ainsi que de nombreuses maladies inflammatoires et autoimmunes, est maintenant reconnu. Il a donc été proposé d'utiliser des produits possédant une activité SOD pour le traitement de ces pathologies. Comme l'administration de SOD pose certains problèmes (notamment de biodisponibilité, et d'effets secondaires résultant de l'immunogénicité de la molécule), ces produits sont le plus souvent des mimétiques non peptidiques de SOD.

L'utilisation de mimétiques de SOD a ainsi été proposée dans le cadre de diverses pathologies impliquant l'anion superoxyde ou ses métabolites [pour revue cf. PATEL et DAY, Trends Pharmacol. Sci., 20, 359-364 (1999)]. Leur efficacité thérapeutique potentielle *in vivo* a jusqu'à présent été étudiée principalement sur des modèles animaux de lésions de reperfusion après ischémie, ou d'inflammation induite par l'anion superoxyde produit par les polynucléaires neutrophiles, [cf. par exemple SALVEMINI et al. : Br. J. Pharmacol., 127, 685-692, (1999) ; Science, 286, 304-306, (1999).

Les mimétiques de la SOD sont généralement des dérivés macrocycliques azotés chélatant un métal, qui est le manganèse dans le cas des mimétiques de la MnSOD. Parmi ceux dont l'activité mimétique de la SOD a été le mieux caractérisée *in vivo,* on citera en particulier des dérivés de métalloporphyrine [PASTERNACK et al., Inorg. Biochem., 15, 261-267 (1981)], tels que le MnTBAP (Mn(III) tetrakis (5,10,15,20-benzoic acid) porphyrin), ou des dérivés macrocycliques tels que ceux décrits dans le Brevet US 5,874,421 au nom de RILEY et al., ou dans la publication de WEISS et al., [J. Biol. Chem, 271, 26149-26156 (1996), qui proposent leur utilisation dans le traitement de pathologies résultant des effets toxiques des radicaux oxygénés libres.

Dans le cas des pathologies hépatiques, les quelques expérimentations rapportées ont porté sur la CuZnSOD ou ses mimétiques. La Demande EP 0333490 rapporte ainsi que les atteintes hépatiques induites par l'administration d'acétaminophène ou de galactosamine sont diminuées par l'administration simultanée de CuZnSOD. MIESEL et al. [Gen. Pharmac., 26, 1261-1266, 1995) rapportent que les atteintes hépatiques résultant de l'administration de galactosamine-LPS sont diminuées par l'administration préalable de CuPu(Py)2, qui est un mimétique non peptidique de la CuZnSOD.

Les Inventeurs ont dans un premier temps recherché l'effet de mimétiques de la SOD sur l'insuffisance hépatique aiguë d'origine toxique, en utilisant un modèle expérimental d'insuffisance hépatique aiguë induite par administration d'acétaminophène. Ils ont observé que l'administration d'un mimétique de la MnSOD, le MnTBAP, permet d'augmenter très significativement le taux de survie après administration d'une dose létale d'acétaminophène, et d'en réduire considérablement les effets toxiques, alors que dans les mêmes conditions, l'administration d'un mimétique de la CuZnSOD, n'a que peu ou pas d'effets.

En outre, ils ont constaté que les effets bénéfiques du MnTBAP sont observés non seulement lorsque celui-ci est administré à titre préventif, mais également lorsqu'il est administré à titre curatif, c'est à dire après l'apparition des premiers effets hépatotoxiques.

Les Inventeurs ont recherché si un effet similaire était observé sur l'insuffisance hépatocellulaire d'origine virale, en utilisant un modèle expérimental d'hépatite fulminante résultant d'une apoptose Fas-dépendante induite par l'administration d'anticorps anti-Fas possédant une activité similaire à celle du FasL. Ils ont constaté que de manière très surprenante, les effets bénéfiques du MnTBAP sur la diminution des lésions hépatiques et le taux de survie des animaux sont encore plus importants que dans le cas d'une insuffisance hépatique aiguë d'origine toxique.

Les Inventeurs ont également recherché les effets du MnTBAP sur l'insuffisance hépatocellulaire d'origine toxique pouvant être induite notamment par l'alcool, en utilisant un modèle expérimental d'intoxication par la diméthylnitrosamine (DMNA) administrée à la dose de 10 mg/Kg chez la souris. Ce modèle se rapproche de l'intoxication par l'alcool car les lésions induites par la DMNA et l'alcool sont identiques.

Ils ont constaté sur des coupes histologiques effectuées à différents temps de l'intoxication, que le MnTBAP retarde l'apparition des lésions hépatiques induites par la DMNA.

La présente invention a pour objet l'utilisation du MnTBAP pour l'obtention d'un médicament destiné au traitement préventif ou curatif d'une insuffisance hépatocellulaire.

Le MnTBAP posséde également d'autres activités intervenant dans la détoxication d'espèces oxygénées réactives autres que l'anion superoxyde. Il est connu pour posséder, outre son activité SOD, une activité catalase. De plus les Inventeurs ont constaté qu'il possédait en outre une activité glutathion peroxydase, qui participe, comme l'activité catalase, à la détoxication de l'eau oxygénée, ce qui peut être avantageux, par exemple, dans le cadre du traitement de l'insuffisance hépatique aiguë d'origine toxique.

Selon un mode de réalisation préféré de la présente invention, le MnTBAP est utilisé pour l'obtention d'un médicament destiné au traitement préventif ou curatif d'une insuffisance hépatocellulaire d'origine toxique, et notamment au traitement d'une insuffisance hépatocellulaire induite par l'acétaminophène, ou d'une insuffisance hépatocellulaire induite par l'alcool.

Selon un autre mode de réalisation préféré de la présente invention, le MnTBAP est utilisé pour l'obtention d'un médicament destiné au traitement préventif ou curatif d'une insuffisance hépatocellulaire résultant d'une apoptose Fas-dépendante, d'une apoptose des hépatocytes médiée par des récepteurs de mort, et en particulier au traitement d'une insuffisance hépatocellulaire d'origine virale.

De manière particulièrement avantageuse, le MnTBAP peut être utilisé pour l'obtention d'un médicament destiné au traitement d'une insuffisance hépatocellulaire aiguë, se manifestant notamment sous forme d'une hépatite fulminante.

L'utilisation, conformément à l'invention, du MnTBAP permet en outre, du fait de la protection apportée vis-à-vis des lésions tissulaires résultant de la destruction des hépatocytes, de prévenir la constitution des lésions fibrotiques pouvant résulter de la cicatrisation de ces lésions.

Pour la mise en oeuvre de la présente invention, le MnTBAP peut être utilisé dans les formulations et les voies d'administrations habituelles pour ce type de composés, telles que celles décrites par exemple dans le Brevet US 5 874 421.

Avantageusement, il sera utilisé dans le cadre de formulations permettant l'administration d'une dose de principe actif comprise entre 0,1 et 10 mg/kg/jour (administration de façon préventive) ou entre 5 et 50 mg/kg/jour (administration de façon curative) ; il est bien entendu toutefois que l'homme de l'art peut adapter ces doses en fonction notamment de l'âge, du poids et de la pathologie du patient.

Ce composé pourra être administré par voie orale, par inhalation, par voie rectale, par voie cutanée ou par voie générale, en particulier par injections sous-cutanées, intra-musculaires ou intra-veineuses, selon la formulation ou la forme galénique souhaitée. D'autres voies d'administration pourront être envisagées si elles augmentent l'efficacité, la biodisponibilité ou la tolérance des produits.

La présente invention sera mieux comprise à l'aide du complément de description qui va suivre, qui se réfère à des exemples non-limitatifs démontrant l'activité du MnTBAP sur l'insuffisance hépato-cellulaire de différentes origines.

### EXEMPLE 1 : ACTIVITE DU MnTBAP SUR L'INSUFFISANCE HEPATIQUE AIGUË INDUITE PAR L'ACETAMINOPHENE.

L'injection intrapéritonéale d'acétaminophène chez la souris induit une hépatotoxicité sévère, dont le degré peut être évalué par la survie des animaux, la mesure des activités transaminase, et l'examen macroscopique et microscopique des foies.

### Survie des animaux

Le MnTBAP, (commercialisé par ALEXIS BIOCHEMICALS) est administré sous forme de bolus, par voie intrapéritonéale.

L'acétaminophène en solution à 100 mg/ml dans du PBS à pH 7,4 est administré par voie intrapéritonéale.

Dans une première série d'expérimentations, un groupe de souris a reçu une dose de 1000 mg/kg d'acétaminophène ; un second groupe a reçu une dose de 1000mg/kg d'acétaminophène, et une dose de 10 mg/kg de MnTBAP administrée soit 2 h avant l'acétaminophène, soit 6 h après ; un groupe témoin a reçu soit du MnTBAP seul (10 mg/kg), soit du PBS seul.

La survie des animaux est suivie pendant 24 heures après l'administration d'acétaminophène.

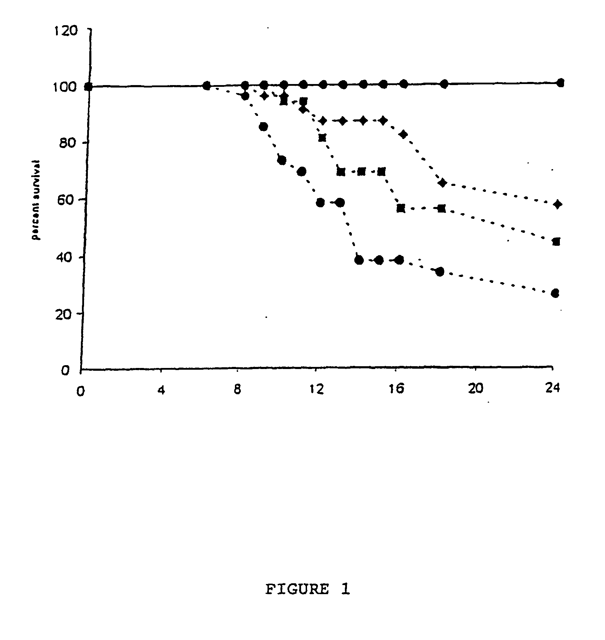

Les résultats sont illustrés par la Figure 1, qui représente le pourcentage de survie en fonction du temps. Ces résultats montrent que 24 heures après l'injection d'acétaminophène, 74% des animaux n'ayant pas reçu de MnTBAP (●) sont morts, alors que le taux de survie est supérieur à 40% chez les animaux ayant reçu le MnTBAP après l'administration d'acétaminophène (■), et de l'ordre de 60% chez les animaux ayant reçu le MnTBAP avant l'administration d'acétaminophène (◆). Aucun décès n'est observé chez les animaux ayant reçu le MnTBAP ou le PBS seuls.

### Dosage des transaminases

Dans une première série d'expérimentations, un groupe de souris a reçu une dose de 1000 mg/kg d'acétaminophène ; un second groupe a reçu la même dose d'acétaminophène, et une dose de 10 mg/kg de MnTBAP administrée 2 h avant l'acétaminophène ; un groupe témoin a reçu soit du MnTBAP seul (10 mg/kg), soit du PBS seul.

Les transaminases sériques ALAT et ASAT sont dosées 12 heures et 24 heures après l'administration d'acétaminophène.

Dans la mesure où les résultats risquaient d'être biaisés par le fait que les souris survivantes 24 heures après l'administration d'acétaminophène étaient probablement, quel que soit le groupe concerné, celles qui présentaient l'activité transaminase la plus faible, une seconde série d'expérimentation a été effectuée, en administrant 500 mg/kg d'acétaminophène, les autres conditions expérimentales demeurant les mêmes. A ce dosage, toutes les souris sont encore en vie après 24 heures.

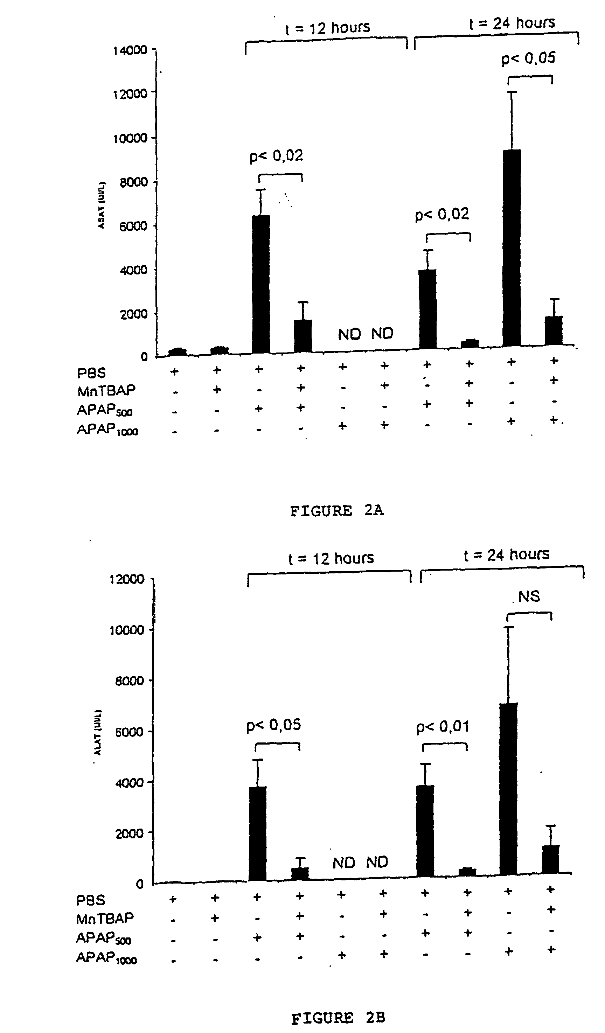

Les résultats sont illustrées par les Figures 2A et 2B, qui représentent respectivement l'activité ASAT et ALAT selon les produits administrés.

Parmi les souris ayant reçu 1000 mg/kg d'acétaminophène (APAP₁₀₀₀), on observe, après 24 heures, des activités transaminases 6 fois moindres chez celles qui ont reçu préalablement un traitement par le MnTBAP. Parmi les souris ayant reçu 500 mg/kg d'acétaminophène (APAP₅₀₀), on observe, après 24 heures, des activités transaminases 10 fois moindres chez celles qui ont reçu préalablement un traitement par le MnTBAP.

Ces résultats montrent que dans tous les cas, l'administration de MnTBAP réduit les activités transaminases, qui reflètent la cytolyse hépatique.

### Etude histologique

Dans chacun des groupes, les foies de plusieurs animaux ont été prélevés, pour effectuer une étude histologique. Dans le cas des souris ayant reçu de l'acétaminophène on observe beaucoup moins de lésions apoptotiques chez les animaux traités au MnTBAP que chez les animaux non traités. Aucune lésion apoptotique n'est visible chez les souris du groupe témoin n'ayant pas reçu d'acétaminophène.

### EXEMPLE 2 : ACTIVITE DU MnTBAP SUR L'INSUFFISANCE HEPATIQUE AIGUË INDUITE PAR DES ANTICORPS ANTI-FAS.

Des anticorps anti-Fas possédant une activité similaire à celle du FasL, sont utilisés dans des modèles expérimentaux d'apoptose. L'injection de ces anticorps à des souris provoque une hépatite fulminante due à une apoptose massive des hépatocytes, entraînant la mort des souris dans les quelques heures suivant l'injection [OGASAWARA et al.,Nature, 364, pp. 806-809, (1993) ; NAGATA, Prog. Mol. Subcell. Biol., 16, pp. 87-103, (1996)].

27 souris contrôle ont reçu, par injection intraveineuse, 6 µg d'un anticorps monoclonal anti-Fas (clone JO2 ; PHARMINGEN) dilués dans 100 µl de sérum physiologique ; un second groupe de 15 souris a reçu le même traitement, précédé par l'administration, 2 heures auparavant, de 10 mg/kg de MnTBAP, comme décrit à l'exemple 1 ci-dessus ; un groupe témoin a reçu du MnTBAP seul (10 mg/kg).

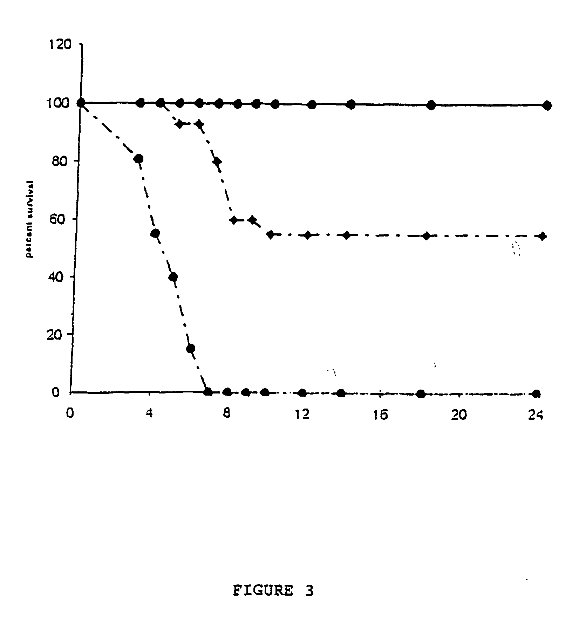

Les résultats sont illustrés par la Figure 3, qui représente le pourcentage de survie en fonction du temps. Ces résultats montrent que 7 heures après l'injection de l'anticorps anti-Fas, la totalité des souris n'ayant pas reçu de MnTBAP (●) sont mortes, alors que le taux de survie à 24 heures est de l'ordre de 60% chez les animaux ayant préalablement reçu le MnTBAP (◆).

### EXEMPLE 3 : ACTIVITE DU MnTBAP SUR L'INSUFFISANCE HEPATO-CELLULAIRE INDUITE PAR UNE INTOXICATION CHRONIQUE PAR LA DMNA.

L'administration intra-péritonéale de 10 mg/kg/jour de DMNA trois fois par semaine, induit chez le chien et le rongeur des lésions centro-lobulaires et péri-portales comportant une fibrose à la 4^{ème} semaine, et une cirrhose à la 13^{ème} semaine [RISTELLI et al., J. Biochem., 158, 361-367, (1976) ; MADDEN et al., Surgery, 68, 260-267, (1970)]. L'administration de MnTBAP par voie intra-péritonéale à la dose de 10 mg/kg, 24 heures après chaque administration de DMNA, prévient la constitution de la fibrose objectivable à l'examen histologique après coloration HES, GORDON, MASSON et PAS.

### EXEMPLE 4 : COMPARAISON DES ACTIVITES DU MnTBAP, DE LA N-ACETYL-L-CYSTEINE, ET DU CuDIPS SUR L'INSUFFISANCE HEPATIQUE AIGUË INDUITE PAR L'ACETAMINOPHENE.

Le CuDIPS (Cu[II]-[diisopropylsalicylate]2 est un mimétique de référence de la CuZnSOD (MC KENZIE et al., Br. J. Pharmacol. 127, 1159-1164, 1999). L'effet de la N-acétyl-L-cystéine (NAC), du CuDIPS, et du MnTBAP sur la survie de souris après injection intrapéritonéale de 1000 mg/kg d'acétaminophène (APAP) a été comparé.

Le protocole expérimental suivi est le même que celui décrit dans l'exemple ci-dessus.

Le MnTBAP, la NAc, ou le CuDIPS sont administrés sous forme de bolus, à titre préventif, 2 heures avant (P) ou à titre curatif, 6 heures après (C) l'acétaminophène.

Différents groupes d'animaux ont recu les traitements suivants :
Groupe I : PBS
Groupe II : MnTBAP 10 mg/kg
Groupe III : NAC 300 mg/kg
Groupe IV : APAP 1000 mg/kg
Groupe V : APAP 1000 mg/kg ; MntBAP 10 mg/kg (P)
Groupe VI : APAP 1000 mg/kg ; MnTBAP 20 mg/kg (P)
Groupe VII : APAP 1000 mg/kg ; MnTBAP 10 mg/kg (C)
Groupe VIII : APAP 1000 mg/kg : MnTBAP 20 mg/kg (C)
Groupe IX : APAP 1000 mg/kg ; MnTBAP 50 mg/kg (P) per os
Groupe X : APAP 1000 mg/kg ; NAC 100 mg/kg (P)
Groupe XI : APAP 1000 mg/kg ; NAC 200 mg/kg (P)
Groupe XII : APAP 1000 mg/kg ; NAC 300 mg/kg (P)
Groupe XIII : APAP 1000 mg/kg ; NAC 100 mg/kg (C)
Groupe XIV : APAP 1000 mg/kg ; NAC 300 mg/kg (C)
Groupe XV : APAP 1000 mg/kg ; CuDIPS 10 mg/kg (P)

La survie des animaux est suivie pendant 24 heures après l'administration d'acétaminophène.

Les résultats, exprimés en pourcentage d'animaux survivants sont illustrés par le tableau I ci après.

Ces résultats montrent que :
- le MnTBAP administré préventivement augmente le taux de survie de manière au moins égale à la NAC ;
- le MnTBAP est actif lorsqu'il est administré par voie orale ;
- contrairement à la NAC, qui n'est active qu'à titre préventif, le MnTBAP est actif lorsqu'il est administré à titre curatif ;
- le CuDIPS n'augmente pas le taux de survie de manière significative.

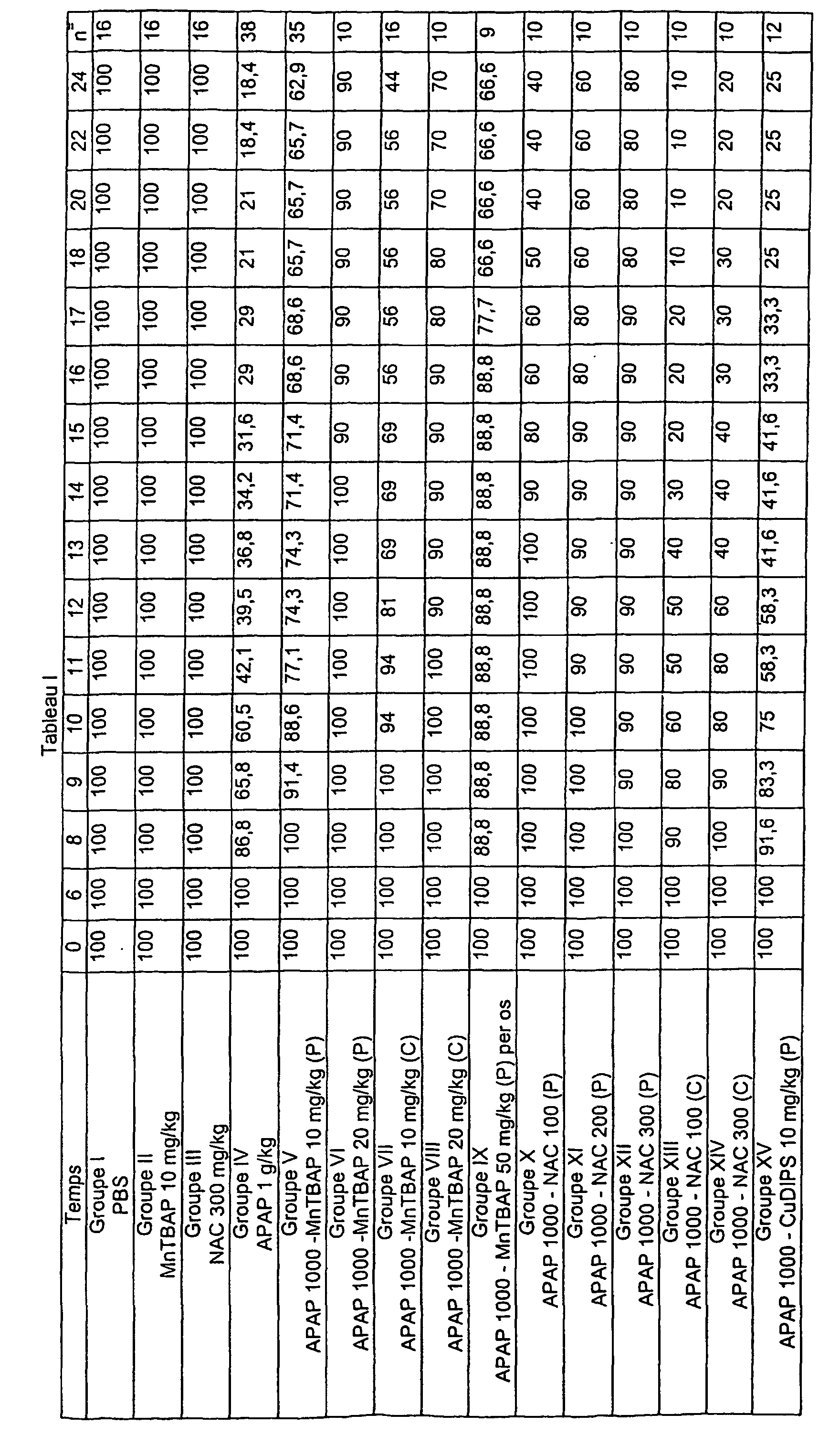

## Revendications

1. Utilisation du MnTBAP [Mn(III) tetrakis (5,10,15,20-benzoic acid) porphyrin] pour l'obtention d'un médicament destiné au traitement préventif ou curatif d'une insuffisance hépatocellulaire.

2. Utilisation selon la revendication 1, **caractérisée en ce que** le MnTBAP est utilisé pour l'obtention d'un médicament destiné au traitement préventif ou curatif d'une insuffisance hépatocellulaire d'origine toxique.

3. Utilisation selon la revendication 2, **caractérisée en ce que** le MnTBAP est utilisé pour l'obtention d'un médicament destiné au traitement préventif ou curatif d'une insuffisance hépatocellulaire induite par l'acétaminophène.

4. Utilisation selon la revendication 1, **caractérisée en ce que** le MnTBAP est utilisé pour l'obtention d'un médicament destiné au traitement préventif ou curatif d'une insuffisance hépatocellulaire résultant d'une apoptose Fas-dépendante des hépatocytes.

5. Utilisation selon la revendication 4, **caractérisée en ce que** le MnTBAP est utilisé pour l'obtention d'un médicament destiné au traitement préventif ou curatif d'une insuffisance hépatocellulaire d'origine virale.

6. Utilisation selon une quelconque des revendications 1 à 2, **caractérisée en ce que** le MnTBAP est utilisé pour l'obtention d'un médicament destiné au traitement préventif ou curatif d'une insuffisance hépatocellulaire induite par l'alcool.

7. Utilisation selon une quelconque des revendications 1 à 6, **caractérisée en ce que** le MnTBAP est utilisé pour l'obtention d'un médicament destiné au traitement d'une insuffisance hépatocellulaire se manifestant sous forme d'une hépatite fulminante.

8. Utilisation selon une quelconque des revendications 1 à 7, **caractérisée en ce que** le MnTBAP est utilisé pour l'obtention d'un médicament destiné à un traitement préventif.

9. Utilisation selon une quelconque des revendications 1 à 7, **caractérisée en ce que** le MnTBAP est utilisé pour l'obtention d'un médicament destiné à un traitement curatif.

## Claims

1. Use of MnTBAP [Mn(III) tetrakis (5,10,15,20-benzoic acid) porphyrin] for obtaining a medication intended for the preventive or curative treatment of hepatocellular insufficiency.

2. Use according to claim 1, **characterised in that** MnTBAP is used for obtaining a medication intended for the preventive or curative treatment of a hepatocellular insufficiency of toxic origin.

3. Use according to claim 2, **characterised in that** MnTBAP is used for obtaining a medication intended for the preventive or curative treatment of a hepatocellular insufficiency caused by acetaminophen.

4. Use according to claim 1, **characterised in that** MnTBAP is used for obtaining a medication intended for the preventive or curative treatment of a hepatocellular insufficiency resulting from an Fas-dependent hepatocyte apoptosis.

5. Use according to claim 4, **characterised in that** MnTBAP is used for obtaining a medication intended for the preventive or curative treatment of a hepatocellular insufficiency of viral origin.

6. Use according to either one of claims 1 to 2, **characterised in that** MnTBAP is used for obtaining a medication intended for the preventive or curative treatment of a hepatocellular insufficiency caused by alcohol.

7. Use according to any one of claims 1 to 6, **characterised in that** MnTBAP is used for obtaining a medication intended for the treatment of a hepatocellular insufficiency manifesting itself in the form of fulminant hepatitis.

8. Use according to any of claims 1 to 7, **characterised in that** MnTBAP is used for obtaining a medication intended for preventive treatment.

9. Use according to any one of claims 1 to 7, **characterised in that** MnTBAP is used for obtaining a medication intended for curative treatment.

## Patentansprüche

1. Verwendung von MnTBAP [Mn(III)Tetrakis(5,10,15,20-benzoesäure)porphyrin] für die Herstellung eines Medikamentes, welches für die Präventiv- oder Heilbehandlung einer hepatozellulären Insuffizienz bestimmt ist.

2. Verwendung nach Anspruch 1, **dadurch gekennzeichnet, daß** das MnTBAP für die Herstellung eines Medikamentes verwendet wird, welches für die Präventiv- oder Heilbehandlung einer hepatozellulären Insuffizienz toxischen Ursprungs bestimmt ist.

3. Verwendung nach Anspruch 2, **dadurch gekennzeichnet, daß** das MnTBAP für die Herstellung eines Medikamentes verwendet wird, welches für die Präventiv- oder Heilbehandlung einer durch Acetaminophen induzierten hepatozellulären Insuffizienz bestimmt ist.

4. Verwendung nach Anspruch 1, **dadurch gekennzeichnet, daß** das MnTBAP für die Herstellung eines Medikamentes verwendet wird, welches für die Präventiv- oder Heilbehandlung einer aus einer Fas-abhängigen Apoptose der Hepatozyten resultierenden hepatozellulären Insuffizienz bestimmt ist.

5. Verwendung nach Anspruch 4, **dadurch gekennzeichnet, daß** das MnTBAP für die Herstellung eines Medikamentes verwendet wird, welches für die Präventiv- oder Heilbehandlung einer hepatozellulären Insuffizienz viralen Ursprungs bestimmt ist.

6. Verwendung nach einem der Ansprüche 1 bis 2, **dadurch gekennzeichnet, daß** das MnTBAP für die Herstellung eines Medikamentes verwendet wird, welches für die Präventiv- oder Heilbehandlung einer durch Alkohol induzierten hepatozellulären Insuffizienz bestimmt ist.

7. Verwendung nach einem der Ansprüche 1 bis 6, **dadurch gekennzeichnet, daß** das MnTBAP für die Herstellung eines Medikamentes verwendet wird, welches für die Behandlung einer in Form einer fulminanten Hepatitis auftretenden hepatozellu-lären Insuffizienz bestimmt ist.

8. Verwendung nach einem der Ansprüche 1 bis 7, **dadurch gekennzeichnet, daß** das MnTBAP für die Herstellung eines Medikamentes verwendet wird, welches für eine Präventivbehandlung bestimmt ist.

9. Verwendung nach einem der Ansprüche 1 bis 7, **dadurch gekennzeichnet, daß** das MnTBAP für die Herstellung eines Medikamentes verwendet wird, welches für eine Heilbehandlung bestimmt ist.
